(19) Europäisches Patentamt
European Patent Office
Office européen des brevets

(11) Veröffentlichungsnummer: **0 164 058 B1**

(12) **EUROPÄISCHE PATENTSCHRIFT**

(45) Veröffentlichungstag der Patentschrift: **02.10.91**

(51) Int. Cl.^5: **C11D 17/00**, C11D 1/52, C11D 1/72, A61K 7/075, A61K 7/50

(21) Anmeldenummer: **85106562.3**

(22) Anmeldetag: **29.05.85**

# (54) Fliessfähige Perlglanzdispersion mit niedrigem Tensidanteil.

(30) Priorität: **07.06.84 DE 3421161**

(43) Veröffentlichungstag der Anmeldung:
**11.12.85 Patentblatt 85/50**

(45) Bekanntmachung des Hinweises auf die Patenterteilung:
**02.10.91 Patentblatt 91/40**

(84) Benannte Vertragsstaaten:
**BE DE FR GB IT NL**

(56) Entgegenhaltungen:
**DE-A- 1 669 152**
**DE-A- 2 016 439**
**DE-A- 3 317 909**

**PATENT ABSTRACTS OF JAPAN, unexamined applications, Sektion C, Band 5, Nr. 136, 28. August 1981 THE PATENT OFFICE JAPANESE GOVERNMENT Seite 108 C 69**

(73) Patentinhaber: **HOECHST AKTIENGESELLSCHAFT**
**Postfach 80 03 20**
**W-6230 Frankfurt am Main 80(DE)**

(72) Erfinder: **Quack, Jochen Meinhard, Dr.**
**Wilhelm-Reuter-Strasse 16**
**W-6239 Eppstein/Taunus(DE)**
Erfinder: **Reng, Alwin**
**Im Schulzehnten 22**
**W-6233 Kelkheim (Taunus)(DE)**
Erfinder: **Skrypzak, Werner**
**Eichkopfallee 33**
**W-6237 Liederbach(DE)**
Erfinder: **Kunz, Walter**
**Karl-Staib-Strasse 21**
**W-6234 Hattersheim am Main(DE)**

**EP 0 164 058 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Bei der Herstellung von kosmetischen Haar- und Körperreinigungsmitteln, Geschirrspülmitteln und flüssigen Wasch- und Reinigungsmitteln werden zur Verbesserung des optischen Aspektes und damit zur Steigerung des Handelswertes, häufig Substanzen verwendet, die den genannten Präparaten ein perlglanzartiges Aussehen verleihen. Um einen solchen Perlglanz- bzw. Seidenglanzeffekt zu erreichen, sind verschiedene Substanzen bekannt, beispielsweise pulverförmige Naturstoffe wie Glimmer, Fischsilber, anorganische Materialien wie Wismutoxichlorid und Titandioxidpigmente, ferner Metallsalze höherer Fettsäuren, Fettsäureglykolester und Fettsäurealkanolamide im Gemisch mit anderen Tensiden.

In letzter Zeit wird zu diesem Zweck häufig ein Fettsäureglykolester allein oder in Kombination mit einem Fettsäurealkanolamid verwendet. Bei diesen beiden Materialien handelt es sich um feste, nichtkristalline Substanzen. Bei der Herstellung von kosmetischen Wasch- und Reinigungsmitteln mit Perlglanzeffekt ist ein Erwärmen des Ansatzes über den Schmelzpunkt der erwähnten Substanzen erforderlich. Hierbei wird eine homogene Schmelze erhalten, aus der nach dem Abkühlen durch Kristallisation ein Perl- bzw. Seidenglanzeffekt entsteht. Dieses Verfahren besitzt jedoch den Nachteil, daß hierbei der gesamte Produktionsansatz auf eine entsprechende Temperatur (je nach Perlglanzbildner ca. 60-80 °C) erhitzt werden muß und erst durch definierte Abkühl- und Rührbedingungen ein mehr oder minder gleichmäßig konstanter Perlglanz erzeugt wird. Hierbei ist ein hoher Energie- und Zeitaufwand erforderlich.

Wärme- bzw. hitzeempfindliche Substanzen wie etherische Öle, Parfümöle und spezielle Wirkstoffe können erst nach Beendigung des Abkühlvorganges zugegeben werden. Die Möglichkeit der kontinuierlichen Herstellung der genannten kosmetischen Präparate mit Perlglanzeffekt ist mit diesem Verfahren ebenfalls nicht gegeben.

Aufgrund dieser beschriebenen Nachteile haben sich in jüngster Zeit Verfahren etabliert, die es ermöglichen, kosmetische Haar- und Körperreinigungsmittel mit Perlglanzeffekt auch bei Raumtemperatur herzustellen. Bei diesen Verfahren werden sogenannte Perlglanzdispersionen, basierend auf den verschiedenartigsten Perlglanzbildnern in Kombination mit Tensiden eingesetzt. Bei der Herstellung dieser Perlglanzdispersionen wird die perlglanzgebende Substanz in hoher Konzentration oberhalb der Schmelztemperatur in das Tensid, das ebenfalls in höherer Konzentration vorliegt, eingearbeitet. Durch konstante, exakt abgestimmte Abkühl- und Rührbedingungen werden hierbei Perlglanzkristalle mit einer definierten Teilchengrößenverteilung gebildet.

Die derzeit am häufigsten verwendeten Substanzen zur Herstellung dieser Perlglanzdispersionen sind Fettsäuremono- und/oder-polyglykolester, die allein oder zusammen mit Fettsäurealkanolamiden in wäßrige Lösungen von Alkylsulfaten oder Polyoxialkylenalkylsulfaten (Alkylethersulfaten) als Lösemittel bzw. Dispergiermittel eingearbeitet werden. In den meisten Fällen werden hierbei sehr hochviskose, bei tieferen Temperaturen nicht mehr fließfähige Dispersionen erhalten.

Aufgrund des hohen Anteiles von Alkylsulfaten bzw. Alkylethersulfaten ist es nicht möglich, mit diesen Perlglanzdispersionen sogenannte alkylsulfat- bzw. alkylethersulfatfreie Kosmetikpräparate herzustellen. Wegen des Dioxangehaltes in Alkylethersulfaten enthalten die mit diesen Perlglanzdispersionen hergestellten Fertigpräparate auch einen gewissen Anteil an Dioxan.

Um die beschriebenen Nachteile der auf dem Markt befindlichen Perlglanzdispersionen auf Basis Alkylethersulfat zu vermeiden, wurden umfangreiche Untersuchungen durchgeführt. Überraschend wurde gefunden, daß bei Verwendung von Fettsäuremono- und/oder polyglykolestern in Kombination mit Fettsäurealkanolamiden und speziellen ethersulfatfreien Tensiden als Netz- bzw. Dispergiermittel Perlglanzdispersionen erhalten werden, die neben einem ausgezeichneten Perlglanzeffekt und einer guten Lagerstabilität sowie einer niedrigen Viskosität, die oben genannten Nachteile nicht aufweisen. Der Tensidanteil dieser Dispersionen kann unter 2 % betragen. Die erfindungsgemäße, fließfähige Perlglanzdispersion besteht aus folgenden Komponenten:

5-30 Gew.-% eines Fettsäureglykolesters der allgemeinen Formel I

$$R_1-\overset{\displaystyle O}{\overset{\|}{C}}-(O-A)_m-O-X \qquad (I)$$

wobei $R_1$ eine lineare oder verzweigte, gesättigte oder ungesättigte Kohlenwasserstoffkette mit 13-21 Kohlenstoffatomen, A eine Gruppe der Formel $-C_2H_4-$ oder $-C_3H_6-$, vorzugsweise $-C_2H_4-$,
X ein Wasserstoffatom oder eine Gruppe der allgemeinen Formel

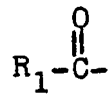

und m eine Zahl von 1-10, vorzugsweise 1-3 bedeuten,
2-20 gew.-% eines Fettsäurealkanolamides der allgemeinen Formel II

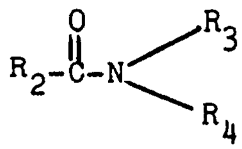 (II)

wobei $R_2$ eine lineare oder verzweigte, gesättigte oder ungesättigte Kohlenwasserstoffkette mit 7-29 Kohlenstoffatomen, $R_3$ und $R_4$ unabhängig voneinander ein Wasserstoffatom oder eine Gruppe der Formel $-C_2H_4OH$ oder $-C_3H_6OH$ bedeuten,
0,1 - 10 Gew.-% eines oder mehrerer Tenside der nachstehend genannten allgemeinen Formel

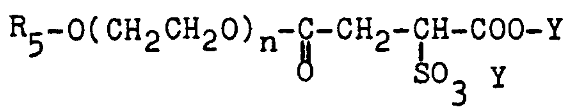 (III)

wobei $R_5$ eine linare oder verzweigte, gesättigte oder ungesättigte Kohlenwasserstoffgruppe mit 8-20 Kohlenstoffatomen, n eine Zahl von 0-10, vorzugsweise 2-5 und Y ein Alkalimetall-, Erdalkalimetall- oder Ammoniumion bedeuten,

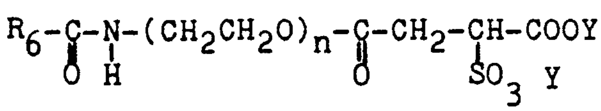 (IV)

wobei $R_6$ eine lineare oder verzweigte, gesättigte oder ungesättigte Kohlenwasserstoffkette mit 7 - 29 Kohlenstoffatomen, n und Y die gleiche Bedeutung haben wie in Formel III,

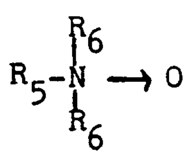 (V)

wobei $R_5$ und $R_6$ die gleiche Bedeutung haben wie in den allgemeinen Formeln III und IV,

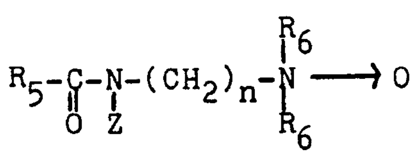 (VI)

wobei $R_5$, $R_6$ und n die gleichen Bedeutungen haben wie in den allgemeinen Formeln III und IV, Z ein Wasserstoffatom oder eine Alkylgruppe mit 1 - 5 Kohlenstoffatomen bedeuten, 0,1 bis 3 %, vorzugsweise 0,5 bis 1 % eines ein- oder zweiwertigen Metallsalzes, vorzugsweise Alkalichlorid oder -sulfat sowie Wasser in der an 100 % fehlenden Menge.

Im Rahmen der oben genannten Einsatzkonzentrationen der einzelnen Komponenten werden unter Einhaltung der nachstehend beschriebenen Herstellbedingungen optimale Perlglanzdispersionen erhalten,

die gegenüber den zur Zeit auf dem Markt angebotenen Perlglanzdispersionen folgende Vorteile aufweisen:

a) ausgezeichnetes Fließverhalten auch bei tieferen Temperaturen (kleiner +15° C). Hierdurch ist die Möglichkeit der kontinuierlichen Verarbeitung gegeben;

b) sehr niedriger Tensidanteil (kleiner 2,0 %). Dadurch keine Belastung der Endformulierung durch ein bestimmtes evtl. unerwünschtes Tensid;

c) frei von Alkylethersulfaten

d) weitgehende Erniedrigung des Dioxangehaltes.

Zur Herstellung der erfindungsgemäßen Perlglanzdispersionen mit optimalen anwendungstechnischen Eigenschaften werden folgende Komponenten in den nachfolgend beschriebenen Einsatzkonzentrationen benötigt.

Als Perlglanzbildner werden Fettsäureglykolester der allgemeinen Formel I sowie Mischungen dieser Substanzklasse verwendet. Die günstigsten Eigenschaften zeigen Verbindungen in denen $R_1$ ein Alkylrest mit 15-17 Kohlenstoffatomen m = 1 und x ein Rest der allgemeinen Formel $R_1$-CO- ist.

Die Einsatzkonzentration dieser Komponente beträgt vorzugsweise 10 - 20 Gew.-%, wobei die optimale Konzentration in der erfindungsgemäßen Perlglanzdispersion mit 16 % ermittelt wurde.

Bei den Fettsäurealkanolamiden gemäß der allgemeinen Formel II werden vorzugsweise solche verwendet, die in der Ausgangsfettsäure eine Verteilung von 8 - 18 Kohlenstoffatomen besitzen. Bevorzugt werden hierbei Fettsäuremonoethanolamide verwendet, wobei einer der beiden Reste $R_3$ oder $R_4$ der allgemeinen Formel II ein Wasserstoffatom und der andere Rest eine Gruppe der Formel $-C_2H_4OH-$ darstellt. Die Einsatzkonzentration dieser Komponente beträgt vorzugsweise 2 - 10 %, insbesondere 4 %.

Unter den genannten Tensiden bzw. Tensidmischungen der allgemeinen Formeln III bis VI werden vorzugsweise Mischungen der Verbindungen mit der allgemeinen Formel III und V verwendet, wobei die Alkylkette $R_5$ 10-18 Kohlenstoffatome, m vorzugsweise 4 und y ein Natriumion bedeuten. Die Einsatzkonzentration dieser beiden Verbindungen beträgt vorzugsweise je 0,9 Gew.-%. Der Gesamtgehalt an Verbindungen der Formeln III bis VI liegt vorzugsweise unter 2 %.

Zum Herstellen der erfindungsgemäßen Perlglanzdispersionen wird wie folgt verfahren.
In einem heizbaren Kessel werden die beiden Komponenten A und B vorgelegt und unter Erhitzen auf ca. 75° C geschmolzen. In diese Schmelze wird uner Rühren eine ebenfalls auf 75° C erhitzte wässrige Lösung der Komponenten C, D und E zugegben. Die Rührgeschwindigkeit sollte nicht zu hoch gewählt werden und in etwa zwischen 10 - 100 Upmin betragen. Die gebildete Dispersion wird bei einer Temperatur von 75° C noch ca. 30 min. gerührt und anschließend unter Rühren bis zu einer Endtemperatur von 20 - 30° C abgekühlt. Die Geschwindigkeit der Abkühlung sollte nach exakt definierten Bedingungen erfolgen, nämlich in der gleichen Weise, wie es auch bei den bisher gebräuchlichen Perlglanzdispersionen gemacht wird, um einen konstanten reproduzierbaren Perlglanzeffekt zu erreichen. Während des Abkühlvorganges beginnen die beiden Komponenten A und B bei ca. 50 - 60° C zu kristallisieren und bilden den gewünschten Perlglanz. Neben den genannten Hauptkomponenten kann die vorliegende Perlglanzdispersion noch Zusätze wie Konservierungsmittel und Puffersubstanzen enthalten, sowie Salze, wie beispielsweise bis zu 3 Gew.-%, vorzugsweise 0,5 bis 1 Gew.-% Natriumchlorid. Der pH-Wert der Dispersion liegt im Bereich von 4 - 9 , vorzugsweise bei 5-8. Um eine mikrobielle Kontamination zu vermeiden, wird der Perlglanzdispersion ein geeignetes Konservierungsmittel zugesetzt.

Die vorliegende, erfindungsgemäße Perlglanzdispersion kann bei Raumtemperatur flüssigen Haar- und Körperreinigungsmitteln, flüssigen Geschirrspülmitteln sowie flüssigen Wasch- und Reinigungsmitteln zugesetzt werden. Hierdurch werden Fertigprodukte erhalten, die einen ausgezeichneten Perlglanz aufweisen. Die hierzu benötigte Menge der Perlglanzdispersion liegt zwischen 1 und 10 %, vorzugsweise 2 - 5 %. Da die erfindungsgemäße Perlglanzdispersion bei Temperaturen über +10° C eine niedrige Viskosität aufweist, besteht die Möglichkeit, die Dispersion mit Hilfe von automatischen Pump-, Dosier- und Mischanlagen zu verarbeiten. Von besonderem Interesse ist dies bei der vollkontinuierlichen Herstellung von perlglanzhaltigen Fertigprodukten.

Die Herstellung der erfindungsgemäßen Perlglanzdispersion wird an folgenden Beispielen erläutert. Die Mengenangaben beziehen sich jeweils auf Gewichtsprozente. Die Herstellung erfolgte in allen Fällen auf die oben beschriebene Art und Weise.

| Beispiel 1: | |
|---|---|
| Zusammensetzung | |
| Monoethylenglykoldistearat | 16,0 % |
| Cocosfettsäuremonoethanolamid | 4,0 % |
| Lauryltriglykolethersulfosuccinat-Natriumsalz | 0,9 % |
| Cocosalkyldimethylaminoxid | 0,9 % |
| Natriumsulfat | 0,6 % |
| Wasser, Konservierungsmittel | ad 100,0 % |

| Beispiel 2: | |
|---|---|
| Zusammensetzung: | |
| Monoethylenglykoldistearat | 10,0 % |
| Cocosfettsäuremonoethanolamid | 10,0 % |
| Lauryltriglykolethersulfosuccinat-Natriumsalz | 0,9 % |
| Cocosalkyldimethylaminoxid | 0,9 % |
| Natriumchlorid | 0,6 % |
| Wasser, Konservierungsmittel | ad 100,0 % |

| Beispiel 3: | |
|---|---|
| Zusammensetzung | |
| Monoethylenglykoldistearat | 16,0 % |
| Cocosfettsäuremonoethanolamid | 4,0 % |
| Lauryltriglykolethersulfosuccinat-Natriumsalz | 0,9 % |
| Alkyldimethylaminoxid | 0,9 % |
| Natriumchlorid | 0,6 % |
| Wasser, Konservierungsmittel | ad 100,0 % |

| Beispiel 4: | |
|---|---|
| Zusammensetzung: | |
| Triethylenglykoldistearat | 16,0 % |
| Cocosfettsäuremonoethanolamid | 4,0 % |
| Lauryltriglykolethersulfosuccinat-Natriumsalz | 0,9 % |
| Cocosalkyldimethylaminoxid | 0,9 % |
| Natriumchlorid | 0,6 % |
| Wasser, Konservierungsmittel | ad 100,0 % |

| Beispiel 5: | |
|---|---|
| Zusammensetzung: | |
| Monoethylenglykolmonostearat | 15 % |
| Stearinfettsäuremonoethanolamid | 5 % |
| Lauryltriglykolethersulfosuccinat-Natriumsalz | 0,9 % |
| Cocosalkyldimethylaminoxid | 0,9 % |
| Natriumsulfat | 0,6 % |
| Wasser, Konservierungsmittel | ad 100,0 % |

| Beispiel 6: | |
|---|---|
| Zusammensetzung: | |
| Triethylenglykol-di-stearat | 16 % |
| Myristinfettsäuremonoethanolamid | 4 % |
| Cocosalkylamido-triethylenglykolethersulfosuccinat-Natriumsalz | 1 % |
| Lauryldimethylaminoxid | 1 % |
| Natriumchlorid | 0,4 % |
| Wasser, Konservierungsmittel | ad 100,0 % |

| Beispiel 7: | |
|---|---|
| Zusammensetzung: | |
| Monoethylenglykolmonostearat | 15 % |
| Myristinfettsäuremonoethanolamid | 5 % |
| Lauryltriethylenglykolethersulfosuccinat-Natriumsalz | 0,9 % |
| Cocosalkylamidoäthylendimethylaminoxid | 1,2 % |
| Natriumchlorid | 0,8 % |
| Wasser, Konservierungsmittel | ad 100,0 % |

Die Perlglanzdispersionen entsprechend den Beispielen 1-4 wurden nach folgenden Kriterien geprüft:

1. Beurteilung des Perlglanzeffektes
2. Bestimmung der sogenannten "optischen Dichte"
3. Messung der Viskosität bzw. des Fließverhaltens
4. Überprüfung der Homogenität
5. Prüfung der Lagerstabilität bei höheren (40 °C) und tieferen Temperaturen als Raumtemperatur.

Als Prüfmethoden wurden die üblicher Weise in der Kosmetik-Industrie verwendeten Testmethoden herangezogen. Die Beurteilung des Perlglanzbildes erfolgt visuell im Vergleich zu den auf dem Markt befindlichen Perlglanzdispersionen. Die Beurteilung der optischen Dichte erfolgt photometrisch in einer Verdünnung von 0,5 g/l Wasser mit Hilfe eines Trübungsmeßgerätes nach Dr. Lange. Unter Berücksichtigung dieser Prüfkriterien zeigen die in den Beispielen 1-4 beschriebenen Perlglanzdispersionen ausgezeichnete, mit handelsüblichen Produkten vergleichbare Eigenschaften. Gegenüber diesen handelsüblichen Dispersionen sind die erfindungsgemäßen Dispersioen aber bereits unter 20 °C fließfähig.

## Patentansprüche

**1.** Fließfähige Perlglanzdispersionen, dadurch gekennzeichnet, daß sie im wesentlichen bestehen aus 5 - 30 Gew.-% eines Fettsäureglykolesters der allgemeinen Formel I

$$R_1-\overset{\overset{\displaystyle O}{\|}}{C}-(O-A)_m-O-X \qquad (I)$$

wobei $R_1$ eine gesättigte oder ungesättigte Kohlenwasserstoffkette mit 13 - 21 Kohlenstoffatomen, A eine Gruppe der Formel $-C_2H_4-$ oder $-C_3H_6-$, vorzugsweise $-C_2H_4-$, X ein Wasserstoffatom oder eine Gruppe der allgemeinen Formel

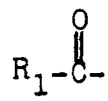

und m eine Zahl von 1-10, vorzugsweise 1-3 bedeuten, 2-20 Gew.-% eines Fettsäurealkanolamides der allgemeinen Formel II

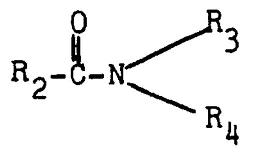

(II)

wobei $R_2$ eine gesättigte oder ungesättigte Kohlenwasserstoffkette mit 7-29 Kohlenstoffatomen, $R_3$ und $R_4$ unabhängig voneinander ein Wasserstoffatom oder eine Gruppe der Formel $-C_2H_4OH$ oder $-C_3H_6OH$ bedeuten, 0,1 - 10 Gew.-% eines oder mehrerer der nachfolgend genannten Tenside der allgemeinen Formeln III - VI bedeuten

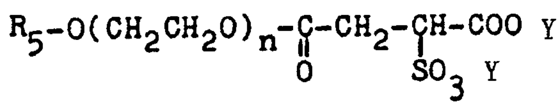

(III)

wobei $R_5$ eine linare oder verzweigte, gesättigte oder ungesättigte Kohlenwasserstoffgruppe mit 8-20 Kohlenstoffatomen, n eine Zahl von 0-10, vorzugsweise 2-5 und Y ein Alkalimetall-, Erdalkalimetall- oder Ammoniumion bedeuten,

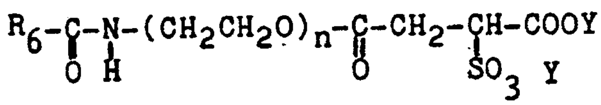

(IV)

wobei $R_6$ eine lineare oder verzweigte, gesättigte oder ungesättigte Kohlenwasserstoffkette mit 7 - 29 Kohlenstoffatomen, n und Y die gleiche Bedeutung haben wie in Formel III,

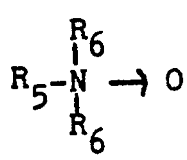

(V)

wobei $R_5$ und $R_6$ die gleiche Bedeutung haben wie in den Formeln III und IV ,

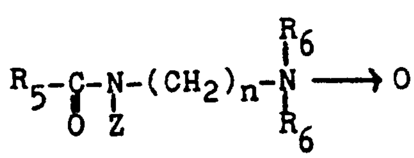

(VI)

wobei $R_5$, $R_6$ und n die gleichen Bedeutungen haben wie in den Formeln III und IV, Z ein Wasserstoffatom oder eine Alkylgruppe mit 1 - 5 Kohlenstoffatomen bedeuten, 0,1 bis 3 %, vorzugsweise 0,5 bis 1 % eines ein- oder zweiwertigen Metallsalzes, vorzugsweise Alkalichlorid oder -sulfat, sowie

Wasser in der an 100 % fehlenden Menge.

## Claims

1. A pearlescent dispersion having good flow properties, which essentially consist of 5 - 30% by weight of a fatty acid glycol ester of the formula I

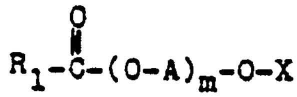

(I)

in which $R_1$ denotes a saturated or unsaturated hydrocarbon chain with 13 - 21 carbon atoms, A denotes a group of the formula $-C_2H_4-$ or $-C_3H_6-$, preferably $-C_2H_4-$, X denotes a hydrogen atom or a group of the formula

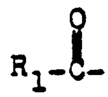

and m denotes a number from 1 to 10, preferably 1 to 3, 2-20% by weight of a fatty acid alkanolamide of the general formula II

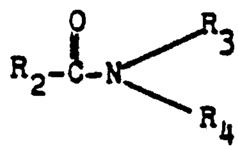

(II)

in which $R_2$ denotes a saturated or unsaturated hydrocarbon chain having 7-29 carbon atoms and $R_3$ and $R_4$ independently of one another denote a hydrogen atom or a group of the formula $-C_2H_4OH$ or $-C_3H_6OH$,
0.1 - 10% by weight of one or more surfactants of the formulae III - VI given below

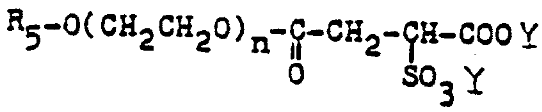

(III)

in which $R_5$ denotes a linear or branched, saturated or unsaturated hydrocarbon group with 8-20 carbon atoms, n denotes a number from 0 to 10, preferably 2 to 5, and Y denotes an alkali metal, alkaline earth metal or ammonium ion,

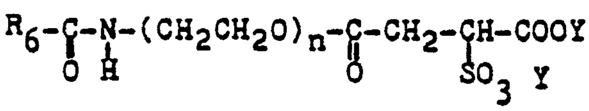

(IV)

in which $R_6$ denotes a linear or branched, saturated or unsaturated hydrocarbon chain having 7 - 29 carbon atoms and n and Y have the same meaning as in formula III,

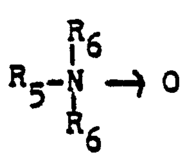

(V)

in which $R_5$ and $R_6$ have the same meaning as in the formulae III and IV, and

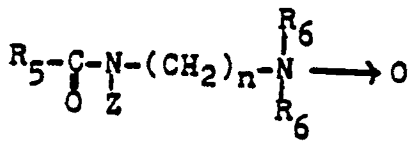 (VI)

in which $R_5$, $R_6$ and n have the same meanings as in the formulae III and IV and Z denotes a hydrogen atom or an alkyl group having 1 - 5 carbon atoms, 0.1 to 3%, preferably 0.5 to 1%, of a monovalent or divalent metal salt, preferably an alkali metal chloride or sulfate, and water in the amount to make up to 100%.

**Revendications**

**1.** Dispersions fluides à éclat nacré, caractérisées en ce qu'elles sont essentiellement constituées :

- de 5 à 30% en poids d'un ester dérivant d'un acide gras et d'un glycol qui répond à la formule générale I :

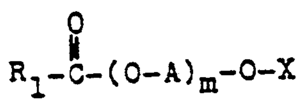 (I)

dans laquelle

$R_1$ représente un radical hydrocarboné saturé ou insaturé, contenant de 13 à 21 atomes de carbone,

A représente un radical $-C_2H_4-$ ou $-C_3H_6-$, de préférence un radical $-C_2H_4-$,

X représente un atome d'hydrogène ou un radical de formule générale :

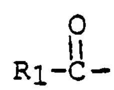

et

m désigne un nombre de 1 à 10, de préférence de 1 à 3,

- de 2 à 20% en poids d'un alcanolamide d'acide gras répondant à la formule générale II :

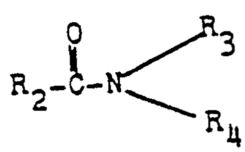 (II)

dans laquelle :

R2 représente un radical hydrocarboné saturé ou insaturé, contenant de 7 à 29 atomes de carbone, et

$R_3$ et $R_4$ représentent chacun, indépendamment l'un de l'autre, un atome d'hydrogène ou un radical $-C_2H_4OH$-ou $-C_3H_6OH-$,

- de 0,1 à 10% en poids d'un ou plusieurs des surfactifs répondant aux formules générales III à VI représentées ci-dessous :

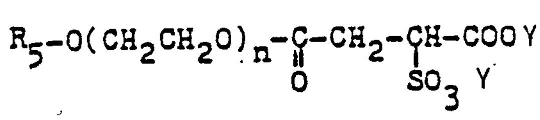 (III)

dans laquelle :

$R_5$ représente un radical hydrocarboné linéaire ou ramifié, saturé ou insaturé, contenant de 8 à 20 atomes de carbone,

n désigne un nombre de 0 à 10, de préférence de 2 à 5, et

Y représente un ion de métal alcalin, de métal alcalinoterreux ou d'ammonium,

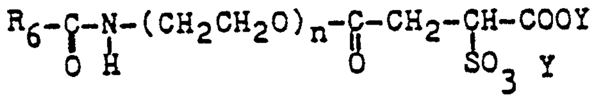

. (IV)

dans laquelle

$R_6$ représente un radical hydrocarboné linéaire ou ramifié, saturé ou insaturé, contenant de 7 à 29 atomes de carbone, et

n et Y ont les mêmes significations que dans la formule III,

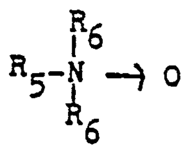

(V)

dans laquelle

$R_4$ et $R_6$ ont les mêmes significations que dans les formules générales III et IV,

et

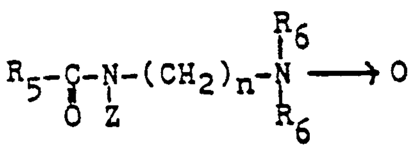

(VI)

dans laquelle

$R_5$, $R_6$ et n ont les mêmes significations que dans les formules générales III et IV, et

Z représente un atome d'hydrogène ou un radical alkyle contenant de 1 à 5 atomes de carbone,

- de 0,1 à 3%, de préférence de 0,5 à 1%, d'un sel métallique uni- ou bivalent, de préférence d'un chlorure ou d'un sulfate de métal alcalin, et
- d'eau en une quantité représentant le complément à 100%.